# EUROPEAN PATENT SPECIFICATION

(11) **EP 1 577 373 B1**
(45) Date of publication and mention of the grant of the patent: **29.11.2006**
(21) Application number: 03814532.2
(22) Date of filing: 25.09.2003
(51) Int. Cl.: C11D 3/33, C11D 1/02, A61Q 19/10, A61K 8/44

(54) **DETERGENT COMPOSITION**
WASCHMITTEL
COMPOSITION DETERGENTE

(30) Priority: 27.12.2002 JP 2002380452
(43) Date of publication of application: 21.09.2005
(73) Proprietor: KAO CORPORATION, Tokyo 103-8210 (JP)
(72) Inventor: SAKURAI, Naoe, c/o KAO CORPORATION RESEARCH LAB., SUMIDA-KU, Tokyo 131-8501 (JP); TAJIMA, Hitoshi, c/o KAO CORPORATION RESEARCH LAB., SUMIDA-KU, Tokyo 131-8501 (JP)
(74) Representative: HOFFMANN EITLE
(86) International application number: PCT/JP2003/012229
(87) International publication number: WO 2004/061071

(56) References cited:
- WO-A-91/18588
- JP-A- 7 258 019
- JP-A- 7 258 699
- JP-A- 9 087 126
- JP-A- 11 130 652
- JP-A- 11 180 855
- JP-A- 57 502 166
- JP-A- 2000 038 593
- US-A- 5 711 942

## Description

### Field of the Invention

The present invention relates to a detergent composition which has excellent stability; moisturizes the skin and therefore gives thereto neither a taut feel nor a dry feel when used for the skin; and enables smooth finger-combing and does not give a rough feel to the hair after shampooing when used for the hair.

### Background of the Invention

For detergent compositions for the skin or hair, anionic surfactants such as higher fatty acid salts, alkyl sulfates, alkylbenzene sulfonates and α-olefin sulfonates have so far been used widely for their high foaming properties. Although these surfactants have high detergency and give the skin a cool and fresh feeling after washing, they leave a taut feel on the skin. As low-irritating surfactants, on the other hand, sulfosuccinate surfactants, ether carboxylate surfactants, amide ether carboxylate surfactants and N-alkylamide alkanol sulfates have been proposed. These surfactants however are not satisfactory in foaming properties and foam quality when used alone.

A skin detergent composition having, in combination, (A') an anionic surfactant and (B') trimethylglycine at a (A')/(B') ratio = 100/1 to 4/1 is proposed as a detergent composition which lathers well and provides an excellent feeling upon use (JP-A-1999-180855). This composition is improved in foaming properties and foam quality, however, it leaves a taut feel on the skin or a rough feel on the hair after washing. For this reason the composition is not satisfactory.

It is known that trimethylglycine reduces skin irritation when incorporated in a cosmetic composition (WO91/18588), or a hair conditioning composition containing trimethylglycine and an aliphatic organic acid (WO82/02337) and a pearly liquid detergent composition containing trimethylglycine and a pearling agent (JP-A-1995-258699) are known.

An object of the present invention is to provide a detergent composition which has excellent stability; moisturizes the skin and therefore gives thereto neither a taut feel nor a dry feel when used as a skin detergent composition; and enables smooth finger-combing and does not give a rough feel to the hair after shampooing when used as a hair detergent.

### Summary of the Invention

The present inventors have found that the above-described problem can be overcome by using an anionic surfactant and trimethylglycine at a specific ratio and adjusting the composition to be weakly acidic.

In the present invention, there is provided a detergent composition comprising (A) an anionic surfactant and (B) trimethylglycine, wherein the components (A) and (B) are added at a weight ratio (A)/(B) = 1/3.5 or greater but less than 4/1 and the pH of the original composition or the pH of the composition after diluted with purified water to a concentration of use is adjusted to 2 or greater but less than 6.5.

### Detailed Description of the Invention

No particular limitation is imposed on the anionic surfactant as the component (A) insofar as it is added to ordinary detergents and can adjust the pH of the composition within the above-described range. Preferred examples include (1) alkyl ether sulfates, (2) alkenyl ether sulfates, (3) ether carboxylate surfactants, (4) amide ether carboxylate surfactants, (5) phosphate surfactants, (6) N-acylamino-acid salt surfactants, (7) polyoxyalkylene fatty acid amide ether sulfates, (8) acylated isethionates, (9) acylated taurates and (10) N-alkylamide alkanol sulfates.

Examples of the alkyl ether sulfates (1) or alkenyl ether sulfates (2) include those having a linear or branched alkyl or alkenyl group with 10 to 20 carbon atoms on average, which is added with 0.5 to 8 moles, on average, of an alkylene oxide per molecule. As the alkylene oxide, ethylene oxide, propylene oxide and butylene oxide can be used either singly or in combination as needed.

As the ether carboxylate surfactants (3) or amide ether carboxylate surfactants (4), those represented by the following formula (1) can be used for example.

R¹-(OC₃H₆)ₐ-(OC₂H₄)_{b}-OCH₂-COOA (1)

(wherein, R¹ represents a linear or branched alkyl or alkenyl group having from 8 to 22 carbon atoms, an alkyl(C₈-C₂₂) phenyl group or a R²CONH-CH₂-CH₂- group (in which R² represents a linear or branched alkyl or alkenyl group having from 11 to 21 carbon atoms), "a" stands for 0 to 6, "b" stands for 2 to 24, and "A" represents an alkali metal, alkaline earth metal or alkanolamine salt residue such as monoethanolamine, diethanolamine or triethanolamine).

As the phosphate surfactant (5), phosphate monoester or diester surfactants having an alkyl or alkenyl group with from 8 to 36 carbon atoms or an alkylene oxide group thereof can be used. Examples include those represented by the following formula (2): 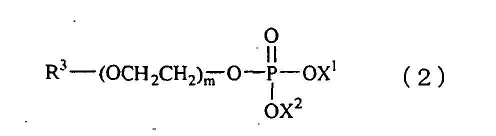 (wherein, R³ represents a hydrocarbon group, X¹ represents a hydrogen atom, an alkali metal, ammonium, a basic amino acid salt, alkanolamine salt, X² represents a hydrogen atom, an alkali metal, ammonium, a basic amino acid salt, an alkanolamine salt or a group R³-(OCH₂-CH₂))ₘ- in which m stands for 0 to 5).

Examples of the hydrocarbon group represented by R³ in the above formula include linear or branched alkyl or alkenyl groups having from 8 to 36 carbon atoms, in which the linear or branched alkyl groups having from 8 to 20 carbon atoms are preferred. Specific examples include octyl, nonyl, decyl, undecyl, dodecyl, tetradecyl, pentadecyl, hexadecyl, octadecyl and icosyl groups.

Examples of the alkali metal as X¹ or X² include lithium, sodium and potassium, those of the basic amino acid include arginine, lysine, histidine and ornithine, those of the alkanolamine include those having a hydroxyalkyl group with from 2 to 3 carbon atoms such as triethanolamine, diethanolamine and monoethanolamine.

In addition, "m" stands for from 0 to 5, in which "m" is preferably 0 to 2.

As such a phosphate surfactant, arginine, potassium or triethanolamine salts of lauryl phosphoric acid, myristyl phosphoric acid, palmityl phosphoric acid or 2-hexyldecyl phosphoric acid are preferred.

As the N-acylamino-acid salt surfactant (6), those having an acyl group with from 8 to 24 carbon atoms are preferred. Specific examples include N-acyl-β-alanine salt, N-acylsarcosine salt and N-acylglutamate salt. Of these, arginine N-lauroyl-β-alaninate, potassium N-lauroyl-β-alaninate, triethanolamine N-lauroyl-β-alaninate, sodium cocoyl glutamate, and sodium N-lauroyl-N-carboxymethyl-β-alaninate are preferred.

Examples of the polyoxyalkylene fatty acid amide ether sulfate salts (7) include those represented by the following formula (3): 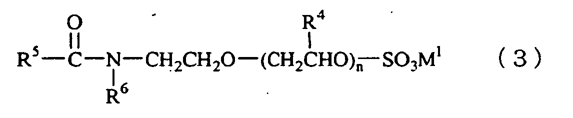 (wherein, R⁴ represents a hydrogen atom or a methyl group, R⁵ represents a linear or branched alkyl or alkenyl group having from 7 to 23 carbon atoms, R⁶ represents a linear or branched alkyl or alkenyl group having from 8 to 24 carbon atoms, n stands for 0 to 20, and M¹ represents a counterion for a sulfuric acid residue).

Examples of the acylated isethionates (8) include acylated isethionates having a linear or branched, saturated or unsaturated fatty acid residue having from 8 to 24 carbon atoms.

Examples of the acylated taurates (9) include acyl taurates having a linear or branched, saturated or unsaturated fatty acid residue having from 8 to 24 carbon atoms.

Examples of the N-alkylamide alkanol sulfates (10) include those represented by the following formula (4): 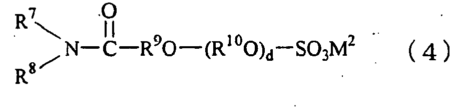 (wherein, R⁷ represents a linear or branched alkyl or alkenyl group having from 6 to 22 carbon atoms, R⁸ represents an alkyl group having from 1 to 22 carbon atoms, an alkenyl group or a hydrogen atom, R⁹ represents a linear or branched alkylene group having from 1 to 5 carbon atoms, R¹⁰O represents an oxyalkylene group having from 2 to 3 carbon atoms, "d" stands for any number of from 0 to 20, "d" pieces of R¹⁰O may be the same or different from one another, and M² represents a hydrogen atom, an alkali metal, an alkaline earth metal, ammonium, an alkanol ammonium having from 2 to 9 carbon atoms in total, an alkyl ammonium or alkenyl ammonium having from 1 to 22 carbon atoms in total, an alkyl or alkenyl-substituted pyridinium having from 1 to 18 carbon atoms, or a basic amino acid).

Examples of the counterion for the anionic residue of these anionic surfactants include alkali metal ions such as sodium and potassium, alkaline earth metal ions such as calcium and magnesium, basic amino acids such as arginine, lysine and histidine, ammonium ions, and alkanolamines having an alkanol group with 2 or 3 carbon atoms, such as monoethanolamine, diethanolamine, triethanolamine and triisopropanolamine.

Anionic surfactants other than the above-described ones may be used in combination insofar as the composition can keep a weakly acidic pH. When a higher fatty acid salt is added, saturated or unsaturated fatty acid salts having from 10 to 24 carbon atoms on average are preferred, but an attention should be paid on selecting a counterion for them. In the case of an alkali metal salt such as sodium or potassium salt, its fatty acid salt is weakly alkaline; therefore it cannot be used for the composition of the present invention at a large amount. In the case of a basic amino acid salt such as lysine, arginine or histidine salt, on the other hand, it is possible to suppress the composition from becoming alkaline even if its fatty acid salt is incorporated and this enables to design the formulation within a preferable pH range. It is therefore preferred to use a basic amino acid salt when a higher fatty acid salt is used in combination.

As the component (A), at least one anionic surfactant can be used and its content in the whole composition is from 0.1 to 40 wt.%, preferably from 0.3 to 30 wt.% from the standpoints of rich lather and a cool and fresh feeling after washing.

Trimethylglycine as the component (B) is an intramolecular salt compound also called "glycine betaine", and commercial product thereof includes "Aminocoat" (product of Asahi Kasei Chemicals Corporation). In the present invention, incorporation of it in an amount of from 0.1 to 40 wt.%, within which the content from 0.5 to 30 wt.% in the whole composition is preferred from the standpoint of feeling upon use after washing.

In the present invention, the components (A) and (B) are added at a (A)/(B) weight ratio = 1/3.5 or greater but less than 4/1, with 1/3 or greater but less than 4/1 being more preferred. When this ratio is less than 1/3.5, the composition has poor stability and the skin after cleansing therewith becomes sticky. When the ratio is 4/1 or greater, on the other hand, the composition cannot sufficiently suppress the skin or hair from becoming dry and rough after cleansing.

The pH of the original detergent composition of the present invention or that after diluted with purified water to a concentration of use is adjusted to from 2 or greater but less than 6.5, preferably from 4 to 6.4. At a pH less than 2, the composition has poor stability, while at a pH of 6.5 or greater, the composition cannot sufficiently suppress the skin or hair from becoming dry and rough after washing. The pH of the composition is adjusted to fall within the above-described range in a conventional manner by using an organic acid or inorganic acid. Examples of the organic acid include citric acid, succinic acid, lactic acid, malic acid, glutamic acid, aspartic acid, pyrrolidonecarboxylic acid, tartaric acid, glycolic acid and ascorbic acid. Examples of the inorganic acid include hydrochloric acid, sulfuric acid and phosphoric acid, with phosphoric acid being preferred.

In the present invention, the pH of a mouth wash or the like which is used at the original concentration is determined by measuring the pH of the stock solution, while the pH of a cleansing agent for hands, body, face, hair or the like which is used by diluting with water is determined by measuring the pH of an aqueous solution (usually, a 5 wt.% aqueous solution) of the composition diluted with purified water to a concentration of use. The pH is measured at 25°C by a pH meter. The pH of a sheet-impregnated type product as described later is also determined either by measuring the pH of the impregnating solution at the original concentration or by measuring after diluting it with purified water (usually as a 5 wt.% aqueous solution).

The detergent composition of the present invention may contain a surfactant other than the above-described one, that is, an amphoteric surfactant, cationic surfactant or nonionic surfactant.

Examples of the amphoteric surfactant include fatty acid amidopropyl betaines, hydroxypropyl sulfobetaine, and desalted secondary imidazolinium betaines.

Examples of the cationic surfactant include linear monoalkyl quaternary ammonium salts having from 12 to 16 carbon atoms and quaternary ammonium salts having a branched alkyl group having from 20 to 28 carbon atoms.

Examples of the nonionic surfactant include amine oxides, monoglycerides, sorbitan fatty acid esters, alkyl saccharides, polyoxyalkylene alkyl ethers, and higher fatty acid alkanolamides. Preferred examples include lauryl dimethylamine oxide, myristyl dimethylamine oxide, monoglyceride isostearate, monoglyceride oleate, monoglyceride octanoate, sorbitan monocaprylate, sorbitan monolaurate, sorbitan monooleate, sorbitan sesquioleate, sorbitan monoisostearate, sorbitan trioleate and coconut oil fatty acid monoethanolamide.

At least one of these amphoteric surfactants, cationic surfactants and nonionic surfactants may be used and their total content in the whole composition is from 0.001 to 30 wt.%, with from 0.01 to 20 wt.% being preferred.

The detergent composition of the present invention may further contain components added to ordinary detergent compositions. Examples include oily components such as lanoline and derivatives thereof, esters such as isopropyl myristate, oil or fats such as coconut oil and olive oil; humectants such as polyglycerin fatty acid esters; bactericides such as triclosan and trichlorocarbanilide; anti-inflammatory agents such as potassium glycyrrhizinate and tocopherol acetate; antiseptics such as methylparaben, ethylparaben, propylparaben and butylparaben; chelating agents such as ethylenediaminetetraacetic acid and salts thereof, and hydroxyethanediphosphonic acid and salts thereof; thickeners such as carboxyvinyl polymer, carrageenan, hydroxyethyl cellulose, and cationic cellulose; polyols such as propylene glycol, 1,3-butanediol, glycerin, sorbitol and maltitol; salts such as sodium chloride; and the other agents such as pearling agent, scrubbing agent, perfume, colorant, ultraviolet absorber, antioxidant and plant extract.

The detergent composition of the present invention can be prepared in a conventional manner by mixing the above-described components. It can be provided in the liquid form, for example, skin detergent compositions such as face wash, makeup remover, body shampoo and hand soap, and hair detergent compositions such as shampoo, or oral detergent compositions such as mouth wash. In addition, sheet-impregnated type of cleansing products such as face-wash sheet and makeup-remover sheet obtained by impregnating a detergent composition of a liquid type in a base material sheet such a woven fabric, nonwoven fabric or paper and then drying the sheet if necessary.

### Examples

### Example 1

The detergent compositions having the composition as shown in Table 1 were prepared in a conventional manner and they were evaluated for non-taut feel, moist feel, non-dry feel, non-sticky feel, smooth finger-combing through the hair and softness of the hair after shampooing, and stability. The results are summarized in Table 1.

In Examples, the pH of the composition was determined by measuring the pH of an aqueous solution obtained by diluting each composition with purified water to 5 wt.% at 25°C by using a Horiba pH meter "F-22" (product of Horiba Ltd.).

### (Evaluation method)

### (1) Non-taut feel, moist feel, non-dry feel, and non-sticky feel of the skin cleansed with the composition:

Organoleptic evaluation of the non-taut feel, moist feel, non-dry feel and non-sticky feel of each detergent composition was made in accordance with the below-described criteria by a panel of ten Japanese female experts in their 20s or 30s after they leathered 1 g of the composition, massaged the whole face with it for 60 seconds, rinsed it with running water and towel dried the face.
3: The face does not become taut, becomes moist, does not become dry, and does not become sticky.
2: The face becomes a little taut, a little moist, a little dry and a little sticky.
1: The face becomes taut, does not become moist, becomes dry and becomes sticky.

Their average score was calculated and the composition is ranked as A, B and C when the average scores are from 2.5 to 3.0, from 1.5 to 2.4, and from 1.0 to 1.4, respectively.

### (2) Smooth finger-combing and softness of the hair after shampooed with the composition:

Organoleptic evaluation of smoothness of finger-combing and softness of the hair of each detergent composition was made in accordance with the below-described criteria by a panel of ten Japanese female experts in their 20s and 30s after they applied 3 g of the composition to their wet hair, shampooed for 120 seconds, rinsed with running water and then dried. Their average score was calculated and each composition was ranked as in (1).
3: The hair can be finger-combed smoothly and is soft.
2: The hair cannot be finger-combed so smoothly and is a little rough.
1: The hair cannot be finger-combed smoothly and is rough.

### (3) Stability:

In a 100 mL glass sample bottle, 80 g of each detergent composition was poured. After it was stored at 5°C for one day, its viscosity was measured and evaluated in accordance with the following criteria:
A: The composition scarcely thickens.
B: A slight thickening is observed.
C: A marked thickening is observed.

**Table 1**

| Components (wt.%) | | Example products | | | | | | Comparative products | | | | | |
|---|---|---|---|---|---|---|---|---|---|---|---|---|---|
| | | 1 | 2 | 3 | 4 | 5 | 6 | 1 | 2 | 3 | 4 | 5 | 6 |
| A | Potassium alkyl (C11,13,15)phosphate | | | 10 | | 15 | 10 | | | 10 | | 15 | 10 |
| | Potassium lauryl phosphate | | | | 10 | | | | | | 10 | | |
| | Sodium polyoxyethylene (2) lauryl ether sulfate | 12 | | | 5 | | 5 | 12 | | | 5 | | 5 |
| | Sodium cocoyl glutamate ("Amisoft CS-22", product of Ajinomoto Takara Corporation) | | 5 | | | | | | 5 | | | | |
| | Sodium N-lauroyl-N-carboxymethyl-β-alaninate | | 5 | | | | | | 5 | | | | |
| B | Trimethylglycine | 5 | 10 | 15 | 4 | 10 | 10 | 5 | 10 | 15 | 4 | 3 | 55 |
| | Glycerin mono-2-ethylhexyl ether | | | 5 | | | | | | 5 | | | |
| | Coconut oil fatty acid monoethanolamide ("Amisol CME", product of Kawaken Fine Chemicals) | 2 | | | | | | 2 | | | | | |
| | Lauric acid amidopropylbetaine | | | 5 | | 5 | 5 | | | 5 | | 5 | 5 |
| | Citric acid | q.s. | q.s. | q.s. | q.s. | q.s. | q.s. | q.s. | q.s. | q.s. | q.s. | q.s. | q.s. |
| | Potassium hydroxide solution | q.s. | q.s. | q.s. | q.s. | q.s. | q.s. | q.s. | q.s. | q.s. | q.s. | q.s. | q.s. |
| | Purified water | Balance | Balance | Bala nce | Balance | Balance | Balance | Balance | Balance | Balance | Balance | Balance | Balance |
| pH (5 wt.%) | | 5.0 | 5.5 | 6.0 | 6.4 | 5.5 | 5.5 | 6.8 | 7.2 | 7.8 | 10.9 | 5.5 | 5.5 |
| (A)/(B) weight ratio | | 12/5 | 1/2 | 2/3 | 15/4 | 3/2 | 3/2 | 12/5 | 1/2 | 2/3 | 15/4 | 5/1 | 3/11 |
| Non-taut feel on the skin after cleansing | | A | A | A | A | A | A | B | B | B | B | B | A |
| Moist feel of the skin after cleansing | | A | A | A | A | A | A | B | B | A | B | B | A |
| Non-dry feel on the skin after cleansing | | A | A | A | A | A | A | C | B | B | C | B | A |
| Non-sticky feel on the skin after cleansing | | A | A | A | A | A | A | A | A | A | A | B | C |
| Smoothness of finger-combing after shampoo | | A | A | A | A | A | A | A | A | B | B | B | B |
| Softness of hair after shampoo | | A | A | A | A | A | A | B | B | C | C | B | A |
| Stability | | A | A | A | A | A | A | B | B | B | B | A | C |

### Example 2 (face wash)

A face wash having the below-described composition was prepared in a conventional manner.

| (Components) | |
|---|---|
| Potassium alkyl(C11,13,15) phosphate | 15 (wt.%) |
| Trimethylglycine | 20 |
| Glycerin mono-2-ethylhexyl ether | 5 |
| Polyoxyethylene (9) tridecyl ether | 5 |
| Cocamidopropylbetaine | 5 |
| Coconut oil fatty acid diethanolamide | 1 |
| Carboxyvinyl polymer | 0.5 |
| Polyethylene glycol ("ALKOX E-100"; product of Meisei Chemical Works) | 0.5 |
| Glycerin | 10 |
| Propylene glycol | 10 |
| Phosphoric acid | q.s. |
| Titanium oxide | 0.01 |
| Methylparaben | 0.2 |
| Perfume | q.s. |
| Dibutylhydroxytoluene | 0.1 |
| Purified water | Balance |

| | |
|---|---|
| (pH (a 5 wt.% aqueous solution) = 5.5, (A)/(B) = 3/4) | |

### Example 3 (face wash)

A face wash having the below-described composition was prepared in a conventional manner.

| (Components) | |
|---|---|
| Sodium polyoxyethylene (2) lauryl ether phosphate | 10 (wt.%) |
| Sodium cocoyl glutamate ("Amisoft CS-11", product of Ajinomoto·Takara Corporation) | 5 |
| Trimethylglycine | 5 |
| Lauroyl hydroxysulfobetaine | 5 |
| Hydroxyethyl cellulose | 0.5 |
| Cationic polymer ("Merquat 550", product of Matsumoto Trading Co.) | 0.5 |
| Ethylene glycol distearate | 3 |
| Sorbitol | 10 |
| Isoprene glycol | 10 |
| Sodium lactate | 1 |
| Mica titanium ("Timiron Starluster MP-115", product of Merck Japan) | 0.01 |
| Sodium benzoate | 0.2 |
| Polyethylene beads | 0.8 |
| 1-Menthol | 0.05 |
| Perfume | q.s. |
| Talc | 3 |
| Disodium Edetate | 0.1 |
| Purified water | Balance |

| | |
|---|---|
| (pH (a 5 wt.% aqueous solution) = 5.0, (A)/(B) = 3/1) | |

### Example 4 (body shampoo)

A body shampoo having the below-described composition was prepared in a conventional manner.

| (Components) | |
|---|---|
| Sodium polyoxyethylene (2) lauryl ether phosphate | 20 (wt.%) |
| Trimethylglycine | 10 |
| Alkyl polyglucoside ("AG-10LK", product of Kao Corp) | 5 |
| Polyoxyethylene (16) lauryl ether | 2 |
| Lauroyl hydroxysulfobetaine | 5 |
| Arginine | 0.5 |
| Hydroxypropylmethyl cellulose | 0.5 |
| Amphoteric polymer ("Merquat plus 3330", product of Matsumoto Trading Co.) | 0.5 |
| Sorbitol | 10 |
| Dipropylene glycol | 10 |
| Malic acid | q.s. |
| 1-Menthol | 0.1 |
| Sodium benzoate | 0.2 |
| Perfume | q.s. |
| Ethylene glycol distearate | 3 |
| 1-Hydroxyethane-1,1-diphosphonic acid ("DEQUEST 2010CS", product of Mitsubishi Monsanto) | 0.1 |
| Colorant (Blue No. 1) | 0.0002 |
| Purified water | Balance |

| | |
|---|---|
| (pH (a 5 wt.% aqueous solution) = 6.4, (A)/(B) = 2/1) | |

### Example 5 (Shampoo)

A shampoo having the below-described composition was prepared in a conventional manner.

| (Components) | |
|---|---|
| Sodium polyoxyethylene (2) lauryl ether sulfate | 15 (wt.%) |
| Trimethylglycine | 5 |
| Coconut oil fatty acid monoethanolamide | 5 |
| Lauric acid amidopropylbetaine | 5 |
| 2-Lauryl-N-carboxymethyl-N-hydroxyethyl-imidazolinium betaine | 5 |
| Serine | 0.5 |
| Polyether-modified silicone ("Silicone SH-3771E", product of Dow Corning Toray Silicone) | 0.5 |
| Dimethylpolysiloxane ("Silicone SH-200C (5000cs)", product of Dow Corning Toray Silicone) | 0.5 |
| Propylene glycol | 10 |
| Ethanol | 1 |
| Silicic anhydride | 0.2 |
| Methylparaben | 0.2 |
| O-[2-Hydroxy-3-(trimethylammonio)propyl]-hydroxyethyl cellulose chloride | 0.1 |
| *Thujopsis dolabrata* extract | 0.1 |
| Perfume | q.s. |
| Citric acid | q.s. |
| Purified water | Balance |

| | |
|---|---|
| (pH (a 5 wt.% aqueous solution) = 5.0, (A)/(B) = 3/1) | |

### Example 6 (hand soap)

A hand soap having the below-described composition was prepared in a conventional manner.

| (Components) | |
|---|---|
| Potassium lauryl phosphate | 5 (wt.%) |
| Trimethylglycine | 2 |
| Coconut oil fatty acid diethanolamide | 3 |
| Polyoxyethylene (4) polyoxypropylene (7) butyl ether | 5 |
| Polyoxyethylene (80) hydrogenated castor oil | 0.5 |
| Lauryl dimethylamine oxide | 5 |
| Cocamidopropylbetaine | 5 |
| Maltitol | 0.5 |
| Glycerin | 10 |
| Ethanol | 1 |
| Tuberose polysaccharide | 0.5 |
| Isopropylmethylphenol ("Biozole", product of Osaka Kasei) | 0.01 |
| Sodium dehydroacetate | 0.2 |
| Perfume | q.s. |
| Purified water | Balance |

| | |
|---|---|
| (pH (a 5 wt.% aqueous solution) = 5.5, (A) / (B) - 5/2) | |

### Example 7 (medicated hand soap)

A medicated hand soap having the below-described composition was prepared in a conventional manner.

| (Components) | |
|---|---|
| Sodium polyoxyethylene (2) lauryl ether sulfate | 10 (wt.%) |
| Trimethylglycine | 20 |
| Alkyl glucoside ("AG-10LK", product of Kao) | 10 |
| Polyoxyethylene (20) coconut oil fatty acid sorbitan ("Rheodol TW-L120", product of Kao) | 1 |
| Polyoxyethylene (60) hydrogenated castor oil | 0.5 |
| Dipotassium glycyrrhizinate | 1 |
| Lauryl trimethylammonium chloride | 10 |
| Glycerin | 10 |
| Ethanol | 1 |
| Perfume | q.s. |
| Colorant (Yellow No. 1) | 0.0001 |
| Disodium Edetate | 0.1 |
| Methylparaben | 0.1 |
| Succinic acid | q.s. |
| Purified water | Balance |

| | |
|---|---|
| (pH (a 5 wt.% aqueous solution) = 4.5, (A)/(B) = 1/2) | |

### Example 8 (mouth wash)

A mouth wash having the below-described composition was prepared in a conventional manner.

| (Components) | |
|---|---|
| Arginine myristyl phosphate | 0.5 (wt.%) |
| Trimethylglycine | 0.5 |
| Ethanol | 5 |
| Sorbitol | 10 |
| Methylparaben | 0.1 |
| Polyoxyethylene/methylpolysiloxane copolymer ("KHF-4", product of Shin-etsu Chemical) | 0.03 |
| Benzethonium chloride | 0.01 |
| Sodium saccharine | 0.001 |
| Perfume, flavor | q.s. |
| Purified water | Balance |

| | |
|---|---|
| (pH (stock solution) = 6.0, (A)/(B) = 1/1) | |

### Example 9 (sheet-impregnated face wash)

A face wash having the below-described composition was prepared in a conventional manner. Cotton nonwoven fabric was impregnated with sufficient amount of prepared face wash, and then dried at 80°C for 1 minute, whereby a dry wipe sheet was obtained. Upon use, it is foamed with water.

| (Components) | |
|---|---|
| Sodium polyoxyethylene (2) lauryl ether sulfate | 20 (wt.%) |
| Trimethylglycine | 10 |
| Cocamidopropylbetaine | 10 |
| Alkyl glucoside ("AG-10LK", product of Kao) | 10 |
| Polyethylene glycol 1500 | 2 |
| 1,3-Butylene glycol | 10 |
| Eucalyptus extract | 0.1 |
| Methylparaben | 0.1 |
| Disodium Edetate | 0.1 |
| 1-menthol | 0.05 |
| Malic acid | q.s. |
| Polyethylene glycol 400 | Balance |

| | |
|---|---|
| (pH (a 5 wt.% aqueous solution) = 5.5, (A)/(B) = 2/1) | |

Detergent compositions obtainable in Examples 2 to 9 each has excellent stability. When cleansed with the skin detergent compositions obtainable in Examples 2 to 4, 6, 7, and 9, the skin becomes moist without a taut feel and in addition, is neither dry nor sticky. When shampooed with the hair detergent composition obtainable in Example 5, the hair can be combed by fingers smoothly and is soft. When a mouth is washed with an intraoral detergent composition obtainable in example 8, the surface of the teeth becomes smooth and a cool and refreshed feeling lasts long.

### Industrial Applicability

The detergent composition of the present invention has excellent stability. After the skin is cleansed with it, it becomes moist without a taut feel, and is neither dry nor sticky. After the hair is shampooed with it, it can be combed smoothly by fingers and is soft. After an oral cavity is washed with it, the surface of the teeth becomes smooth and a cool and fresh feeling lasts long.

## Claims

1. A detergent composition comprising (A) an anionic surfactant and (B) trimethylglycine, wherein the components (A) and (B) are added at a weight ratio (A)/(B) = 1/3.5 or greater but less than 4/1 and the pH of the original composition or the pH of the composition diluted with purified water to a concentration of use is adjusted to 2 or greater but less than 6.5.

2. The detergent composition according to Claim 1, wherein the anionic surfactant as the component (A) is selected from alkyl ether sulfates, alkenyl ether sulfates, ether carboxylate surfactants, amide ether carboxylate surfactants, phosphate surfactants, N-acylamino acid salt surfactants, polyoxyalkylene fatty acid amide ether sulfates, acylated isethionates, acylated taurates and N-alkylamido alkanol sulfates.

3. The detergent composition according to Claim 1 or 2, which contains the components (A) and (B) in amounts of from 0.1 to 40 wt.% and from 0.1 to 40 wt.%, respectively.

4. The detergent composition according to any one of Claims 1 to 3, further comprising a surfactant selected from amphoteric surfactants, cationic surfactants and nonionic surfactants in an amount of from 0.001 to 30 wt.%.

5. The detergent composition according to any one of Claims 1 to 4, which is a skin detergent composition.

6. The detergent composition according to any one of Claims 1 to 4, which is a face wash.

## Patentansprüche

1. Reinigungszusammensetzung, umfassend (A) ein anionisches Tensid und (B) Trimethylglycin, worin die Komponenten (A) und (B) bei einem Gewichtsverhältnis (A)/(B) = 1/3,5 oder mehr, aber weniger als 4/1 zugegeben sind und der pH der ursprünglichen Zusammensetzung oder der pH der Zusammensetzung, die mit gereinigtem Wasser auf eine Konzentration zur Verwendung verdünnt ist, auf 2 oder mehr, aber weniger als 6,5 eingestellt ist.

2. Reinigungszusammensetzung nach Anspruch 1, worin das anionische Tensid als Komponente (A) ausgewählt ist aus Alkylethersulfaten, Alkenylethersulfaten, Ethercarboxylat-Tensiden, Amidethercarboxylat-Tensiden, Phospat-Tensiden, N-Acylaminosäuresalz-Tensiden, Polyoxyakylenfettsäureamidethersulfaten, acylierten Isethionaten, acylierten Tauraten und N-Alkylamidoalkanolsulfaten.

3. Reinigungszusammensetzung nach Anspruch 1 oder 2, die die Komponenten (A) und (B) in Mengen von 0,1 bis 40 Gew.-% bzw. 0,1 bis 40 Gew.-% enthält.

4. Reinigungszusammensetzung nach einem der Ansprüche 1 bis 3, weiterhin umfassend ein Tensid, ausgewählt aus amphoteren. kationischen und nichtionischen Tensiden in einer Menge von 0,001 bis 30 Gew.-%.

5. Reinigungszusammensetzung nach einem der Ansprüche 1 bis 4, die eine Hautreinigungszusammensetzung ist.

6. Reinigungszusammensetzung nach einem der Ansprüche 1 bis 4, die ein Gesichtswaschmittel ist.

## Revendications

1. Composition détergente comprenant (A) un agent tensioactif anionique et (B) une triméthyl glycine, dans laquelle les composants (A) et (B) sont ajoutés dans un rapport pondéral (A)/(B) = 1/3,5 ou supérieur mais inférieur à 4/1, et le pH de la composition originelle ou le pH de la composition diluée avec de l'eau purifiée pour obtenir une concentration d'utilisation est ajusté à 2 ou à une valeur supérieure mais inférieure à 6,5.

2. Composition détergente selon la revendication 1, dans laquelle l'agent tensioactif anionique en tant que composant (A) est choisi parmi les sulfates d'alkyl éther, les sulfates d'alcényléther, les agents tensioactifs d'éther carboxylate, les agents tensioactifs d'amide éther carboxylate, les agents tensioactifs de phosphate, les agents tensioactifs de sel d'acide N-acylaminé, les sulfates d'éther amide d'acide gras polyoxyalkylènés, les isothionates acylés, les taurates acylés et les sulfates de N-alkylamido alkanol.

3. Composition détergente selon la revendication 1 ou 2 qui contient les composants (A) et (B) respectivement dans des quantités de 0,1 à 40 % en poids et de 0,1 à 40 % en poids.

4. Composition détergente selon l'une quelconque des revendications 1 à 3, comprenant de plus un agent tensioactif choisi parmi les agents tensioactifs amphotères, les agents tensioactifs cationiques et les agents tensioactifs non ioniques en quantité de 0,001 à 30 % en poids.

5. Composition détergente selon l'une quelconque des revendications 1 à 4, qui est une composition détergente de la peau.

6. Composition détergente selon l'une quelconque des revendications 1 à 4, qui est un produit de lavage du visage.
